# EUROPÄISCHE PATENTSCHRIFT

(11) **EP 2 686 417 B1**
(45) Veröffentlichungstag und Bekanntmachung des Hinweises auf die Patenterteilung: **08.06.2016**
(21) Anmeldenummer: 12712611.8
(22) Anmeldetag: 19.03.2012
(51) Int. Cl.: C12N 5/00, C12N 5/078

(54) **TCRALPHA/BETA-DEPLETIERTE ZELLPRÄPARATIONEN**
CELL PREPARATIONS DEPLETED OF TCR ALPHA/BETA
PRÉPARATIONS DE CELLULES AVEC DÉPLÉTION EN TCR ALPHA/BÊTA

(30) Priorität: 17.03.2011 DE 102011001380; 07.06.2011 EP 11168949
(43) Veröffentlichungstag der Anmeldung: 22.01.2014
(73) Patentinhaber: Miltenyi Biotec GmbH, 51429 Bergisch Gladbach (DE)
(72) Erfinder: HANDGRETINGER, Rupert, 72076 Tübingen (DE); HUPPERT, Volker, 51515 Kürten (DE)
(74) Vertreter: Hoppe, Georg Johannes
(86) Internationale Anmeldenummer: PCT/EP2012/054805
(87) Internationale Veröffentlichungsnummer: WO 2012/123590

(56) Entgegenhaltungen:
- BETHGE ET AL: "Haploidentical allogeneic hematopoietic cell transplantation in adults using CD3/CD19 depletion and reduced intensity conditioning: An update", BLOOD CELLS, MOLECULES AND DISEASES, LAJOLLA, US, Bd. 40, Nr. 1, 5. Dezember 2007 (2007-12-05), Seiten 13-19, XP022376430, ISSN: 1079-9796, DOI: 10.1016/J.BCMD.2007.07.001
- BARFIELD R C; OTTO M; HOUSTON J; HOLLADAY M; GEIGER T; MARTIN J; LEIMIG T; GORDON P; CHEN X; HANDGRETINGER R: "A one-step large-scale method for T- and B-cell depletion of mobilized PBSC for allogeneic transplantation.", CYTOTHERAPY, Bd. 6, Nr. 1, 1. Januar 2004 (2004-01-01), Seiten 1-6, XP9145422, GB ISSN: 1465-3249
- ZINNO FRANCESCO; LANDI FABIOLA; AURELI VIVIANA; BALDUINO GEPPINA; LANTI ALESSANDRO; SODANI PIETRO; ADORNO GASPARE; LUCARELLI GUIDO: "Positive immunomagnetic CD34(+) cell selection in haplo-identical transplants in beta-thalassemia patients: removal of platelets using an automated system", CYTOTHERAPY, Bd. 12, Nr. 1, 1. Januar 2010 (2010-01-01) , Seiten 60-66, XP9160576, GB ISSN: 1465-3249, DOI: 10.3109/14653240903348301
- ISIDORI ET AL: "Positive Selection and Transplantation of Autologous Highly Purified CD133<+> Stem Cells in Resistant/Relapsed Chronic Lymphocytic Leukemia Patients Results in Rapid Hematopoietic Reconstitution without an Adequate Leukemic Cell Purging", BIOLOGY OF BLOOD AND MARROW TRANSPLANTATION, KLUGE CARDEN JENNINGS PUBLISHING, CHARLOTTESVILLE, VA, US, Bd. 13, Nr. 10, 19. September 2007 (2007-09-19), Seiten 1224-1232, XP022260999, ISSN: 1083-8791
- CHALEFF S; OTTO M; BARFIELD R C; LEIMIG T; IYENGAR R; MARTIN J; HOLIDAY M; HOUSTON J; GEIGER T; HUPPERT V; HANDGRETINGER R: "A large-scale method for the selective depletion of alpha beta T lymphocytes from PBSC for allogeneic transplantation", CYTOTHERAPY, Bd. 9, Nr. 8, 1. Januar 2007 (2007-01-01), Seiten 746-754, XP9160686, GB ISSN: 1465-3249, DOI: 10.1080/14653240701644000 in der Anmeldung erwähnt
- DATABASE BIOSIS [Online] BIOSCIENCES INFORMATION SERVICE, PHILADELPHIA, PA, US; April 2011 (2011-04), SCHUMM M ET AL: "Depletion of TcRab+ and CD19-positive cells from leukapheresis products with the CliniMACS device", XP002678955, Database accession no. PREV201100309915 & BONE MARROW TRANSPLANTATION, Bd. 46, Nr. Suppl. 1, April 2011 (2011-04) , Seiten S339-S340, 37TH EUROPEAN GROUP FOR BONE AND MARROW TRANSPLANTATION/27TH MEETING OF THE EBMT NURSES GROUP/10TH M; PARIS, FRANCE; APRIL 03 -06, 2011 ISSN: 0268-3369(print)
- MICHAEL SCHUMM ET AL: 'Depletion of T-cell receptor alpha/beta and CD19 positive cells from apheresis products with the CliniMACS device' CYTOTHERAPY Bd. 15, Nr. 10, 01 Oktober 2013, Seiten 1253 - 1258, XP055173116 DOI: 10.1016/j.jcyt.2013.05.014 ISSN: 1465-3249

## Beschreibung

Die vorliegende Erfindung betrifft TCRalpha/beta (TCRα/β)-depietierten Zellpräparationen, die TCR gamma/delta-positive Zellen aufweisen, sowie deren Herstellung.

### Einleitung

In Europa werden jährlich mehr als 31.000 Stammzelltransplantationen durchgeführt, von denen ca. 13.000 allogen und 18.000 autolog sind (Baldomero H, Gratwohl M, Gratwohl A, Tichelli A, Niederwieser D, Madrigal A, Frauendorfer K. The EBMT activity survey 2009: trends over the past five years. Bone Marrow Transplant. 2011 Feb 28). Sie gewinnt immer mehr an Bedeutung in der Behandlung von hämatologischen, onkologischen, immunologischen und genetischen Erkrankungen (Zintl et al., Correction of fatal genetic diseases using bone marrow transplantation. Kinderarztl Prax. 1991 Jan-Feb; 59(1-2):6-9; Zintl, Bone marrow transplantation in childhood. I. Kinderarztl Prax. 1988 Jun;56(6):259-64; Down JD, Mauch PM. The effect of combining cyclophosphamide with total-body irradiation on donor bone marrow engraftment. Transplantation. 1991 Jun; 51(6):1309-11) und stellt für viele dieser Erkrankungen die einzige langfristige Heilungsmöglichkeit dar (Eyrich et al., A prospective comparison of immune reconstitution in pediatric recipients of positively selected CD34+ peripheral blood stem cells from unrelated donors vs recipients of unmanipulated bone marrow from related donors. Bone Marrow Transplant. 2003 Aug; 32(4):379-90).

Die Hauptkomplikationen der Stammzelltransplantation ergeben sich vor allem aus der Reaktion des Transplantats gegen den Empfänger (Graft-versus-host-disease, GvHD oder GvHR), einem fehlerhaften Engraftment der transplantierten Stammzellen, der Toxizität der Konditionierung und den Infektionen, unter Therapie aufgrund einer verzögerten oder inkompletten Immunrekonstitution.

Mit reduziertem Konditionierungsregime konnten bei allogener Transplantation die Inzidenz von therapieassoziierter Mortalität gesenkt werden. Neben der Reduktion potenzieller chemotherapieassoziierter Nebenwirkungen für den Empfänger wird durch reduzierte Post-Transplant Immunsuppression tendenziell der immunologische Effekt des Transplantats gegen das maligne Gewebe begünstigt. Dieser Transplantat-gegen-Tumor-Effekt wird insbesondere durch T- und NK-Zellen des Spenders vermittelt. Trotz der reduzierten Konditionierung steigt die Tumorprogression nicht an (Valcárcel et al., Conventional versus reduced-intensity conditioning regimen for allogeneic stem cell transplantation in patients with hematological malignancies. Eur J Haematol. 2005 Feb;74(2):144-51; Strahm et al.. Reduced intensity conditioning in unrelated donor transplantation for refractory cytopenia in childhood. Bone Marrow Transplant. 2007 Aug; 40(4): 329-33). Um erhöhte Mortalität zu vermeiden, gilt es also nach allogener Transplantation das Immunsystem so schnell wie möglich wieder herzustellen um eine Kontrolle des Tumors und von Infektionen zu ermöglichen. Dies ist mit dem Auftreten von GvHD zu balancieren, welche bei frühem Absetzen der Immunsuppression, der Verwendung von zu vielen alloreaktiven T-Zellen oder bei zu großer Antigendifferenz begünstigt wird und Mortalität und Morbidität erzeugt

### 1) Passive TCD (CD34 Anreicherung)

Bisher wurden Patientinnen und Patienten, die eine Stammzelltransplantation benötigten, mit einer Zellpräparation aus CD34 positiven Zellen (Stammzellen) als zweckmäßiger Vergleichstherapie behandelt. Auf diese Weise werden T-Zellen depletiert, die bei der GvHD eine maßgebliche Rolle spielen, allerdings gehen mit den T-Zellen, aber auch NK-Zellen auch unabdingbare Faktoren (Effektorzellpopulationen) verloren, die bei der Heilung eine Rolle spielen.

Unter der Graft-versus-Host-Reaktion (GvHR; deutsch: *Transplantat-Wirt-Reaktion;* englisch: Graft-versus-Host-Disease (GvHD)) versteht man eine immunologische Reaktion, welche in der Folge einer allogenen Knochenmark- oder Stammzelltransplantation auftreten kann (Jacobsohn DA, Vogelsang GB: Acute graft versus host disease. Orphanet J Rare Dis. 2007 Sep 4; 2:35).

Bei der GvHR reagieren vor allem die im Transplantat enthaltenen T-Lymphozyten eines Spenders gegen den Empfängerorganismus. Sie ist damit immunologisch gesehen eine Reaktion der Spender-Lymphozyten auf die fremden Antigene des Patienten.

Man unterscheidet eine akute GvHD (aGvHD) und eine chronische GvHD (cGvHD) voneinander, wobei die chronische Form 100 Tage nach Transplantation aus der akuten oder als de-novo GvHD entstehen kann.

Nach dem Seattle Schema werden die akute und chronische Form in Grade unterteilt. An Haut, Darm und Leber manifestiert sich die GvHD mittels Exanthemen und Blasen auf der Körperoberfläche, Diarrhoen, Ileus und angestiegenem Bilirubin. Für die aGvHD ergibt sich aus der Summe der Manifestationsorte und der Schwere der Manifestation eine Unterteilung von Grad 0 bis Grad IV. Um eine aGvHD zu vermeiden, können prophylaktische Maßnahmen eingeleitet werden. Zu diesen zählen die Gabe von Immunsuppressiva wie Methotrexat (MTX), Cyclosporin A (CsA), Kortikosteroiden oder die Kombination aus diesen Medikamenten. (Martino R, Romero P et al.: "Comparison of the classic Glucksberg criteria and the IBMTR Severity Index for grading acute graft-versus-host disease following HLA-identical sibling stem cell transplantation. International Bone Marrow Transplant Registry." Bone Marrow Transplant 1999; 24(3): S. 283-287 und Wikipedia, Graft-versus-Host-Reaktion).

Das Risiko, eine GvHD zu entwickeln hängt eng mit der Kompatibilität ab, welche durch das humane Leukozyten-Antigen (HLA) bestimmt wird. Ein besonders hohes Risiko, eine aGvHD zu erleiden, besteht bei unverwandter Fremdspende und HLA-inkompatibler Spende. Bei allogener Transplantation von HLA-identischen Geschwisterspendern entwickeln jedoch trotz optimaler Vorsorge ca. 35-60 % der Patienten eine akute GvHD leichter bis mittlerer Ausprägung trotz der Verabreichung von immunsupprimierenden Medikamenten; ca. 10 % erleiden eine schwer kontrollierbare GvHD (Kanda Y, Chiba S: "Allogeneic hematopoietic stem cell transplantation from family members other than HLA-identical siblings over the last decade (1991-2000)." Blood 2003; 102(4): S. 1541-1547).

Ein erheblicher Anteil (ca. 30 - 65 %) der Patienten entwickelt eine chronische GvHD, die langfristig eine der häufigsten Nebenwirkungen und Todesursachen nach Stammzelltransplantation darstellt (Ringden et al., The graft-versus-leukemia effect using matched unrelated donors is not superior to HLA-identical siblings for hematopoietic stem cell transplantation. Blood. 2009 Mar 26; 113(13): 3110-8; Ratanatharathorn et al., Phase III study comparing methotrexate and tacrolimus (prograf, FK506) with methotrexate and cyclosporine for graft-versus-host disease prophylaxis after HLA-identical sibling bone marrow transplantation. Blood. 1998 Oct 1;9 2(7): 2303-14.), die Lebensqualität nachhaltig beeinträchtigt, sowie die Rückkehr an den Arbeitsplatz verzögert (Wong et al., Long-term recovery after hematopoietic cell transplantation: predictors of quality-of-life concerns. Blood. 2010 Mar 25;115(12):2508-19.; Sutherland et al., Quality of life following bone marrow transplantation: a comparison of patient reports with population norms. Bone Marrow Transplant. 1997 Jun;19(11):1129-36).

Zusätzlich erfordert nicht nur die Prophylaxe, sondern auch die Behandlung der GvHD die Verabreichung von Immunsuppressiva, die erhebliche Nebenwirkungen verursachen können, z. B. Kortikosteroid-Nebenwirkungen wie Diabetes, avaskuläre Nekrose, Cushing's Syndrom oder CsA/Tacrolimus Nebenwirkungen wie Nierenschädigungen, Bluthochdruck, Paresthesias und allgemein Infektionen jeder Art (Wong et al., Long-term recovery after hematopoietic cell transplantation: predictors of quality-of-life concerns. Blood. 2010 Mar 25; 115(12):2508-19; Sutherland et al., Quality of life following bone marrow transplantation: a comparison of patient reports with population norms. Bone Marrow Transplant. 1997 Jun; 19(11): 1129-36; Ferrara JL, Levine JE, Reddy P, Holler E. Graft-versus-host disease. Lancet. 2009 May 2;373(9674):1550-61).

### Immunrekonstitution

Ein weiterer Nachteil der bisherigen Behandlungsmethode ist die verzögerte Immunrekonstitution, d.h. die verzögerte Wiederherstellung eines funktionierenden Immunsystems bzw. hämatopoietischen Systems im transplantierten Patienten. Das Immunsystem bedarf zur Rekonstitution ca. 1 bis 2 Jahre.

In dieser Zeit besteht das stark erhöhte Risiko, an lebensbedrohlichen Infektionen vor allem viralen, bakteriellen oder Pilzinfektionen zu erkranken bzw. zu versterben (Handgretinger R, Klingebiel T, Lang P et al. Megadose transplantation of purified peripheral blood CD34(+) progenitor cells from HLA-mismatched parental donors in children. Bone Marrow Transplant 2001;27:777-83 und Platzbecker U, Ehninger G, Bornhauser M. Allogeneic transplantation of CD34+ selected hematopoietic cells: clinical problems and current challenges. Leuk Lymphoma 2004;45:447-53).

Die Ursache hierfür liegt in der Tatsache, dass mit der Anreicherung von CD34 positiven Stammzellen sämtliche T-Zellen, NK-Zellen und weitere akzessorische Zellpopulationen verloren gehen, die bei der Immunrekonstitution bzw. der Rekonstitution des hämatopoietischen Systems helfen könnten.

Die Regeneration der T-Zellen nach Transplantation und damit der Immunrekonstitution verläuft auf zwei Wegen. Der sogenannte zentrale Weg ist thymusabhängig und setzt einen intakten Thymus voraus. T-Zellen, die den Thymus erst kürzlich verlassen haben, weisen auf die Erholung des Immunsystems hin. Die Bestimmungen des T-Zell-Rezeptor Exzisions Zyklus (TREC) und unreifer T-Zellen mit dem Oberflächenantigen CD45RA eignen sich zur Darstellung. Der periphere Weg der T-Zell-Rekonstitution ist thymusunabhängig und sehr wichtig, da viele Konditionierungsregime den Thymus schädigen. Die Expansion reifer T-Lymphozyten, welche mit dem Transplantat übertragen werden, gewährleistet die Rekonstitution des Immunsystems.

CD34+selektionierte Transplantationen, bei denen die T-Zellen nicht auf den Patienten übertragen werden, weisen somit ein verspätetes Einsetzen der Rekonstitution des Immunsystems auf (Sutherland et al., Reconstitution of naive T cells and type 1 function after autologous peripheral stem cell transplantation: impact on the relapse of original cancer. Transplantation. 2002; 73: 1336-9; Rutella et al., Immune reconstitution after autologous peripheral blood progenitor cell transplantation: effect of interleukin-15 on T-cell survival and effector functions. Exp Hematol. 2001; 29:1503-16; Heining et al., Lymphocyte reconstitution following allogeneic hematopoietic stem cell transplantation: a retrospective study including 148 patients. Bone Marrow Transplant. 2007; 39: 613-22). Die geringe GvHD Rate bei diesen Transplantationen wird durch eine stark verzögerte Immunrekonstitution sozusagen erkauft. Daraus ergibt sich als aktuelle Schwachstelle die verzögerte Immunrekonstitution d. h. die verzögerte Wiederherstellung eines funktionierenden Immunsystems, die mit einem erhöhten Risiko potentiell letaler Infektionen assoziiert ist.

### Relapse

Ein weiterer Nachteil der T-Zell-Depletion ist das erhöhte Risiko, dass die zugrundeliegende Erkrankung, die Grund für die Stammzelltransplantation war (meist eine Leukämie), nach CD34-Stammzelltransplantationen häufiger wieder auftritt (Horowitz MM, Gale RP, Sondel PM et al. Graft-versus leukemia reactions after bone marrow transplantation. Blood 1990;75:555-62), auch durch die Entfernung der NK-Zellen (natural killer cells), die einen anti-leukämischen Effekt haben (Ruggeri L, Mancusi A, Capanni M et al. Exploitation of alloreactive NK cells in adoptive immunotherapy of cancer, Curr Opin Immunol 2005;17:211-7).

### 2) Aktive TCD (CD3 Depletion)

CD3-depletierte Zellpräparationen wurden kürzlich verwendet, in denen die T-Zellen depletiert sind; NK-Zellen, Monozyten, Granulozyten und CD34 negative Stammzellvorläuferzellen im Transplantat aber noch enthalten sind. Zwar war das Risiko von GvHD und therapieassoziiertem Frühversterben (*early treatment related mortality,* TRM) etwas verkleinert, allerdings führten auch diese Zellpräparationen nicht zu einer messbaren Erhöhung der Überlebensrate (Lee CK, DeMagalhaes-Silverman M et al.: "Donor T-lymphocyte infusion for unrelated allogeneic bone marrow transplantation with CD3+ T-cell-depleted graft." Bone Marrow Transplant 2003; 31(2): S. 121-128 und Wikipedia, Graft-versus-Host-Reaktion) oder einer verbesserten Immunrekonstitution.

Bethge et al. (Haploidentical alloveneic hematopoietic cell transplantation in adults using CD3/CD 19 depletion and reduced intensity conditioning: An update, Blood Cells, Molecules and Diseases, 490, 1, 13-19) beschreibt die Depletion von CD3/CD19-positiven Zellen.

### Zusammenfassung der Erfindung

In einem ersten Aspekt betrifft die Erfindung eine Zusammensetzung, insbesondere eine pharmazeutische Zusammensetzung, die eine aus Knochenmark oder aus Blut erhaltbare Zellpopulation aufweist. Erfindungsgemäß ist die Zellpopulation von TCR (T-Zell Rezeptor) alpha/beta-positiven Zellen depletiert. Es finden sich somit T-Zellen in der (pharmazeutischen) Zusammensetzung, die TCR gamma/delta positiv sind, aber nur noch wenige oder fast keine oder, im besten Fall, keine Zellen mehr, die TCR alpha/beta positiv sind.

Unter Depletion wird hier die signifikante Abreicherung von Zellen aus einer Zellpopulation verstanden. So kann Depletion eine Abreicherung einer Zellart (die durch das Vorhandensein z. B. eines Zelloberflächenmarkers definiert sein kann, wie TCRalpha/beta oder CD19) um mindestens 2 logarithmische Stufen, bevorzugt um mindestens 3 logarithmische Stufen, besonders bevorzugt um mindestens 4 logarithmische Stufen (z. B. 4,6 logarithmische Stufen), am meisten bevorzugt um mindestens 4 bis 5 logarithmische Stufen.

Die Entfernung nach logarithmischen Stufen ist wie folgt: 1 log = 90 % Entfernung der störenden Zellen, 2 log = 99 %, 3 log = 99,9 % und 4 log = 99,99 %. Arten zur Berechnung von Separationsperformance sind dem Fachmann bekannt und z. B. in Bosio et al., Isolation and Enrichment of Stem Cells, Advances in Biochemical Engineering and Biotechnology, Springer Verlag Berlin Heidelberg, 2009, beschrieben.

Eine derartige Depletion erfolgt über den Oberflächenmarker TCR alpha/beta und optional auch über CD19. Die Depletion kann mit jeder im Stand der Technik bekannten Methode erfolgen, z. B. Panning, Elutriation oder magnetischer Zellseparation. Bevorzugt ist eine Depletion mittels magnetischer Zellseparation (z. B. CliniMACS, Miltenyi Biotec GmbH) aufgrund der hohen Depletionseffizienz.

Die aus Blut erhaltbare Zellpopulation ist insbesondere ein Zellpräparat, das mittels einer Leukozytenapherese oder Knochenmarkpunktion erhalten wird. Bevorzugt wird das Zellpräparat von einem gesunden Spender gewonnen, der zuvor mit stammzellmobilisierenden Medikamenten behandelt wurde.

Bevorzugter Weise ist die Zellpopulation dieser (pharmazeutischen) Zusammensetzung auch hinsichtlich CD19-positiven Zellen depletiert. Dies führt dazu, dass die Zellpopulation keine B-Zellen enthält. Damit entfällt eine mögliche Übertragung des Epstein-Barr-Virus (EBV) an den die pharmazeutische Zusammensetzung empfangenden Patienten und es müssen weniger oder keine Immunsuppressiva verabreicht werden.

In einer bevorzugten Ausführungsform der pharmazeutischen Zusammensetzung weist die Zusammensetzung weiterhin mindestens einen pharmazeutisch akzeptablen Träger oder Zusatzstoff auf. Derartige Träger oder Zusatzstoffe sind dem Fachmann bekannt.

Die pharmazeutische Zusammensetzung kann gegen Krebserkrankungen verabreicht werden, wie beispielsweise Leukämien und andere Erkrankungen, z. B. akute myeloide Leukämien, akute lymphatische Leukämien, Agranulocytose, B-Thalassenämie, Inborn Error (HHS) als auch gegen solide Tumoren (z. B. Neuroblastom, Sarkome etc.), bei denen eine allogene Transplantation indiziert ist, oder ein therapeutischer Effekt von TCR alpha/beta depletierten Zellzubereitungen zu erwarten ist.

Weiterhin müssen bei einer allogenen Transplantation genügend CD34+ Zellen übertragen werden (mindestens 2 bis 4 Millionen pro kg Körpergewicht des Empfängers), um eine gute Rekonstitution der Blutbildung zu erreichen und es sollten weniger als 25,000 TCR alpha/beta positive T Zellen pro kg Körpergewicht des Empfängers gegeben werden, wenn auf eine Immunsuppression verzichtet werden soll. B Zellen, welche durch eine CD19 Depletion aus dem Transplantat entfernt werden, sollten so wenig wie möglich enthalten sein, oder nachträglich im Empfänger durch z. B. die Gabe eines anti-CD19 Antikörpers in vivo entfernt werden wenn das Risiko einer EBV Infektion und der damit auftretenden Komplikationen vermindert werden soll.

Die einem menschlichen Patienten zu verabreichende Menge der depletierten Zellpopulation liegt typischer Weise bei 2x10E10 bis 1x10E11 Lymphocyten.

In einem weiteren Aspekt betrifft die Erfindung eine Verwendung einer aus Knochenmark oder aus Blut erhaltbaren Zellpopulation zur Herstellung einer pharmazeutischen Zusammensetzung, wobei die Zellpopulation von TCR alpha/beta-positiven Zellen depletiert ist und TCR gamma/delta-positive Zellen aufweist.

In einem weiteren Aspekt betrifft die Beschreibung eine Verwendung der pharmazeutischen Zusammensetzung zur Rekonstitution des hämatopietischen Systems eines Menschen nach einer Stammzell- und/oder Knochenmarkstransplantation. Diese Rekonstitution ist gegenüber der bisher bekannten Rekonstitution (z. B. mit unverändertem Knochenmark oder CD34 positiven Stammzellen aus Knochenmark oder Blut bzw. mobilisiertem, prozessiertem Blut nach Leukapherese) deutlich schneller und führt damit zu einem geringeren Bedarf an Transfusionen von Blutbestandteilen, der Möglichkeit eines kompletten Verzichts auf, oder einer Reduktion immunsuppremierender Medikamente und führt somit zu geringeren Nebenwirkungen, weniger Infektionen, und einer verringerten Sterblichkeit des Transplantatempfängers.

In einem weiteren Aspekt betrifft die Erfindung ein Verfahren, insbesondere ein in vitro Verfahren, zur Herstellung einer Population von Zellen. Das Verfahren weist die folgenden Schritte auf:
- Bereitstellen von Knochenmark oder Blut eines Spenders (also einer Population, die u. a. TCR alpha/beta positive und TCR gamma/delta positive Zellen aufweist) und
- Depletion von TCR alpha/beta-positiven Zellen aus der Zellpopulation.

Bevorzugter Weise erfolgt in dem Verfahren die Depletion von TCR alpha/beta positiven Zellen mittels eines Antikörpers oder eines Antigen-bindenden Fragments gegen TCR alpha/beta. Anhand einer der Protein- oder Nucleotidsequenzen gemäß SEQ ID NOs 4 bis 14 des Rezeptors TCR alpha/beta (siehe Tabelle 1 und Sequenzprotokoll) kann ein Antikörper oder ein Antigen-bindendes Fragment, oder ein Derivate oder Konjugat davon gegen TCR alpha/beta hergestellt und zur Depletion von TCR alpha/beta-positiven Zellen verwendet werden.

In einer bevorzugten Ausführungsform weist das Verfahren zusätzlich den folgenden Schritt auf:
- Depletion von CD19-positiven Zellen aus der Zellpopulation. Dieser Schritt kann zeitlich, vor, nach, oder gleichzeitig mit der Depletion von TCR alpha/beta-positiven Zellen aus der Zellpopulation durchgeführt werden.

In dem Verfahren kann die Depletion von CD19-positiven Zellen mittels eines Antikörpers oder eines Antigen-bindenden Fragments gegen CD19 erfolgen. Anhand einer der Protein- oder Nukleotidsequenzen (SEQ ID NOs 1 bis 3) des Oberflächenmarkers CD19 (siehe Tabelle 1 und Sequenzprotokoll) kann ein Antikörper oder ein Antigen-bindendes Fragment, oder Derivat oder Konjugat davon gegen CD19 hergestellt und zur Depletion von CD19-positiven Zellen verwendet werden.

Der Stand der Technik erlaubt einem Fachmann in Kenntnis der Protein- oder Nukleotidsequenzen für TCR alpha/beta und CD19 (im Stand der Technik bekannt) einen Antikörper, ein Antigen-bindendes Fragment, oder Derivate oder Konjugate davon mit den bekannten Methoden herstellen (z. B. Köhler, G. & Milstein, C. (1975): Continuous cultures of fused cells secreting antibody of predefined specificity. In: Nature, 256, 495-497; Shirahata S, Katakura Y, Teruya K. (1998): Cell hybridization, hybridomas, and human hybridomas. In: Methods in cell biology, 57, S. 111-145; Cole SP, Campling BG, Atlaw T, Kozbor D, Roder JC. (1984): Human monoclonal antibodies. In: Molecular and cellular biochemistry, 62, S. 109-120).

Tabelle 1: Bezeichnungen und SEQ ID NOs der humanen Protein- bzw. Nukleotidsequenzen des Oberflächenmarkers CD19 und der Untereinheiten des Rezeptors TCR alpha/beta (TCRA/TRBC). Für die Rezeptoruntereinheit TCR beta (TRBC) sind jeweils zwei Protein- und cDNA-Sequenzen angegeben. Die erste Aminosäure in SEQ ID NO 11

"E" (entspricht "GAG" in SEQ ID 13) liegt auf einer Spleiß-Stelle und ist daher möglicher Weise variabel. In der Proteinsequenz SEQ ID 10 und korrespondierend in der Nukleotidsequenz sind die Aminosäure bzw. Nukleotide daher nicht angegeben.

| **SEQ ID NO.** | **Bezeichnung** | **Sequenzart** |
|---|---|---|
| 1 | CD19_Human | Proteinsequenz |
| 2 | CD19_Humane cDNA | Nukleotidsequenz |
| 3 | CD19_Humane genomische Sequenz | Nukleotidsequenz |
| 4 | TCRA_Human | Proteinsequenz |
| 5 | rCRA_Humane CDS | Nukleotidsequenz |
| 6 | TCRA_Humane genomische Sequenz | Nukleotidsequenz |
| 7 | TRBC1_Human | Proteinsequenz |
| 8 | TRBC1_Humane CDS | Nukleotidsequenz |
| 9 | TR8C1_Humane genomische Sequenz | Nukleotidsequenz |
| 10 | TRBC2_Human) | Proteinsequenz |
| 11 | TRBC2_Human_2 | Proteinsequenz |
| 12 | TRBC2_Humane_CDS | Nukleotidsequenz |
| 13 | TRBC2_Humane_CDS_2 | Nukleotidsequenz |
| 14 | TRBC2_Humane genomische Sequenz | Nukleotidsequenz |

In einem weiteren Aspekt betrifft die Beschreibung eine Verwendung eines beschriebenen Verfahrens zur Rekonstitution der Immunabwehr bzw. des hämatopoietischen Systems eines Menschen im Rahmen einer Stammzell- oder Knochenmarkstransplantation.

Die Beschreibung betrifft weiterhin die Verwendung einer aus Knochenmark oder aus Blut erhaltenen Zellpopulation, wobei die Zellpopulation von Zellen, die TCR alpha/beta exprimieren, depletiert ist, nämlich zur Rekonstitution der Immunabwehr eines Menschen im Rahmen einer Knochenmarkstransplantation. Wie oben beschrieben sind bei dieser Verwendung auch CD19 positive Zellen depletiert.

In einem weiteren Aspekt betrifft die Erfindung eine Verwendung eines Antikörpers oder eines Antigen-bindenden Fragments gegen TCR alpha/beta und/oder TCR alpha/ beta und eines Antikörpers oder eines Antigen-bindenden Fragments gegen CD19 zur Herstellung einer Population von Zellen in vitro, die TCR alpha/beta und/oder CD19 depletiert ist und TCR gamma/delta-positive Zellen aufweist.

In einem weiteren Aspekt betrifft die Beschreibung eignen Kit zur Herstellung einer Population von Zellen, die TCR alpha/beta depletiert und/oder CD19 depletiert sind und TCR gamma/deltapositive Zellen aufweist. Ein derartiger Kit weist einen Antikörper oder ein Antigen-bindendes Fragment davon gegen TCR alpha/beta und/oder einen Antikörper gegen CD19 oder ein Antigen-bindendes Fragment davon auf.

In einem weiteren Aspekt betrifft die Erfindung ebenfalls eine Verwendung des beschriebenen Kits zur Herstellung einer Population von Zellen, die TCR alpha/beta negativ und CD19 negativ ist und TCR gamma/delta-positive Zellen aufweist. Die Zellpopulation kann sowohl in vitro vorliegen und für wissenschaftliche Zwecke bestimmt sein oder als pharmazeutische Zusammensetzung vorliegen, ggf. mit einem pharmazeutisch akzeptablen Träger und/oder einem Zusatzstoff.

### Ausführliche Beschreibung der Erfindung

### Vorteile der Erfindung

Trotz der Verbesserungen in den letzten Jahren ist die Stammzelltransplantation immer noch mit einer akut erhöhten Morbidität und einer initialen transplantationsbedingten Mortalität verbunden. Die Hauptkomplikationen der Stammzelltransplantation, ergeben sich vor allem aus den Abstoßungsreaktionen (Graft-versus-Host Erkrankung) und deren Therapie, der langsamen Immunrekonstitution bzw. Rekonstitution des hämatopoietischen Systems und den daraus resultierenden Infektionen nach Transplantation sowie der Toxizität der Konditionierung.

Durch die Verwendung von TCR alpha/beta depletierten Zellen oder TCR alpha/beta und CD19 depletierten Zellen (depletierte Zellpopulation) ist es möglich, das hämatologische Stammzelltransplantat so zu manipulieren, dass die Hauptkomplikationen nach Stammzelltransplantation, d. h. die Graft-versus-host Krankheit, sowie die langsame Immunrekonstitution nach Stammzelltransplantation und die damit verbundene lang andauernde Infektanfälligkeit gegenüber bakteriellen, viralen und Pilzinfektionen mit potentiell letalem Ausgang, erheblich zu reduzieren.

Gleichzeitig bleibt die Graft-versus-Tumor Reaktivität des Zellgemisches erhalten d.h. es ist kein erhöhtes Rückfallrisiko für den Patienten zu erwarten. Ein weiterer Vorteil ist ein stabiles Anwachsen des Transplantats das sogenannte Engraftment.

Durch die erhebliche Reduktion der genannten Hauptkomplikationen ist es möglich, die Medikamente, die üblicherweise zur Behandlung dieser Komplikationen eingesetzt werden und zum Teil erhebliche Nebenwirkungen hervorrufen (und hohe Kosten verursachen), stark zu reduzieren oder vollständig darauf zu verzichten. Durch den Verzicht der medikamentösen Immunsuppressiva zur GvHD-Prophylaxe können Nebenwirkungen wie z. B. Infektionen (Sepsis, Candidose, Herpes simplex, etc.) die aus der medikamentösen Unterdrückung des Immunsystems resultieren, vermieden werden.

Als Konsequenz kann zum einen die Lebensqualität des Patienten, sowie der Therapieerfolg erheblich verbessert und gleichzeitig die Kosten für die Behandlung reduziert werden. Zu den oben genannten Medikamenten gehören Medikamente zur GvHD Prophylaxe wie z. B. Mofetilmycophenolat, das als GvHD Prophylaxe eingesetzt wird.

Neben der Reduktion von Infektionen und der GvHD ermöglicht die depletierte Zellpopulation die Verwendung eines reduzierten Konditionierungsregimes (RIC). Reduzierte Konditionierungsregime können bei allogener Transplantation die Inzidenz von therapieassoziierter Mortalität senken und dann eingesetzt werden, wenn der immunologische Effekt des Transplantats gegen das maligne Gewebe ausgenutzt werden kann. Dieser Transplantat gegen Tumor Effekt wird insbesondere durch T- und NK-Zellen des Spenders vermittelt und ist daher bei CD34 angereicherten Stammzellpräparaten nicht einsetzbar.

Insofern liegt ein erheblicher Zusatznutzen gegenüber der zweckmäßigen Vergleichstherapie(n) vor, da eine nachhaltige und gegenüber der zweckmäßigen Vergleichstherapie bisher nicht erreichte große Verbesserung des therapierelevanten Nutzens erreicht wird, insbesondere eine Heilung der Erkrankung, eine erhebliche Verlängerung der Überlebensdauer, eine langfristige Freiheit von schwerwiegenden Symptomen und die weitgehende Vermeidung schwerwiegender Nebenwirkungen.

Die Nutzung der TCR alpha/beta Depletionsstrategie resultiert in eine frühere Immunrekonstitution, mit Werten von mehr als100 CD4 Zellen/µl innerhalb von 6 Wochen nach der Transplantation verglichen zu 10 Monaten, wie von Aversa et al. Berichtet (Treatment of high-risk acute leukemia with T-cell-depleted stem cells from related donors with one fully mismatched HLA haplotype. N Engl J Med. 1998 Oct 22;339(17):1186-93.)

### Technische Ergebnisse

Durchgeführte Abreicherungsläufe (Depletion runs bzw. Depletion) mit CliniMACS TCRaß-Biotin (Miltenyi Biotec GmbH) belegen, dass die Effizienz der Abreicherung mit einer durchschnittlichen Log Abreicherung von 4,6 sehr robust ist (Figur 1).

CD19 ist ein Oberflächenmolekül auf T-Zellen. CD19 positive Zellen bedeutet, dass ein CD19-bindendes Molekül, z. B. ein Antikörper an das CD19-Molekül auf der Oberfläche der T-Zellen spezifisch binden kann.

TCR alpha-beta ist ein Oberflächenmolekül auf T-Zellen. TCR alpha/beta positive Zellen bedeutet, dass ein TCR alpha/beta-bindendes Molekül, z. B. ein Antikörper, an das TCR alpha/beta-Molekül auf der Oberfläche der T-Zellen spezifisch binden kann.

"Antikörper" bedeutet ein monoklonaler, polyklonaler Antikörper (Harlow and Lane, "Antibodies, A Laboratory Manual", CSH Press, Cold Spring Harbor, USA), der an ein Molekül bindet oder ein Derivat dieses Antikörpers, der die Bindungskapazität behält oder im Wesentlichen behält. Bevorzugte Derivate dieser Antikörper sind chimäre Antikörper umfassend beispielsweise chimäre Antikörper eine variable Region der Maus oder der Ratte und eine humanen konstanten Region. Der Begriff "Antikörper" umfasst ebenfals einen bifunktionalen oder bispezifischen Antikörper und Antikörper-Konstrukte wie Fvs (scFv) aus Einzelketten oder Antikörper-Fusionsproteine. Der Begriff "scFv" (single chain Fv Fragment) ist der Fachperson bekannt und bevorzugt, da das Fragment rekombinant hergestellt werden kann.

Der Antikörper kann human oder humanisiert sein. Der Begriff "humanisierter Antikörper" bedeutet, dass mindestens eine Antikörperbindungsstelle (complementary determining region (CDR) wie z. B. CDR3 und vorzugsweise alle 6 CDRs ausgetauscht wurden durch CDRs von einem humanen Antikörper mit der gewünschten Spezifität. Optional wurden die nicht-humane(n) konstante(n) Region(en) des Antikörpers ersetzt durch a) konstante Region (en) eines humanen Antikörpers. Methoden zur Herstellung von humanisierten Antikörpern sind beschrieben z. B. in EP 0239400 A1 und WO 90/07861 A1.

Der Begriff Antigen-bindendes Fragment bezeichnet ein Fragment des oben definierten Begriffs "Antikörper" wie z. B. getrennte leichte und schwere Ketten, Fab, Fab/c, Fv, Fab', F(ab')2. Ein antigen-bindendes Fragment kann eine variable Region der leichten Kette und eine variable Region der schweren Kette umfassen, nicht notwendigerweise beide zugleich.

### Klinische Ergebnisse

Im Rahmen von Heilversuchen wurden 11 Patienten behandelt. 8 Patienten mit einem TCRαβ depletierten Transplantat von einem haploidenten Spender und 3 Patienten mit einem Transplantat von einem passenden unverwandten Spender (matched unrelated donor).

Aufgrund der sehr robusten Depletion und damit geringen Zellzahl von TCRαβ⁺T-Zellen im Transplantat musste keine GvHD Prophylaxe in Form von immunsupprimierenden Medikamenten nach der Transplantation wie z. B. MMF oder CsA verabreicht werden.

Die Graft-versus-Host Erkrankung war in den behandelten Patienten reduziert. (Figur 2). In allen Fällen war nur die Haut von GvHD Symptomen betroffen und die Symptome traten nur temporär auf. 36 % der Patienten zeigten GvHD Grad I, 18 % zeigten GvHD Grad II. GvHD Grad III wurde nicht beobachtet. Das ist besonders bemerkenswert, da keine GvHD Prophylaxe in Form von immunsupprimierenden Medikamenten nach der Transplantation verabreicht wurden. Bei 10 von 11 Patienten wuchs das Transplantat zwischen Tag 7 und Tag 9 an.

In allen Patienten wurde eine stark beschleunigte Immunrekonstitution beobachtet. Wie in Figur 1 zu sehen ist, war die Immunrekonstitution mit TCRαβ/CD19 depletierten Zellpopulationen (Transplantaten) deutlich schneller als mit CD3/CD19 depletierten Zellen (Transplantaten).

Dargestellt ist die Immunrekonstitution bis zum Erreichen von >200 Zellen/µl nach Transplantation von TCRαβ/CD19 depletierten peripheren Blutstammzellen (N=11) im Vergleich zu CD34 angereicherten peripheren Blutstammzellen (historische Kontrolle, N=13). Die Daten zur Immunrekonstitution nach Stammzelltransplantation mit CD34 angereicherten Zellen wurden der folgenden Publikation entnommen: Br J Haematol. 2001 Aug;114(2):422-32.

In allen diesen Patienten ist eine sehr schnelle Immunrekonstitution zu beobachten, die völlig überraschend ist und bisher nicht erklärbar, da die Rekonstitution auch deutlich schneller erfolgt als bei der Gabe von unmanipuliertem Knochenmark, welches sämtliche immunrekonstituierende Zellen des Spenders enthält und daher bei unmanipuliertem Knochenmark eigentlich eine schnellere Immunrekonstitution zu erwarten gewesen wäre als bei der Verabreichung von TCR alpha/beta und CD19 depletiertem Zellpräparat, welches deutlich weniger immunrekonstituierende Zellen enthält.

Figur 5 zeigt die Immunrekonstitution eines bei Patienten bei drei aufeinanderfolgenden Stammzelltransplantationen. Die erste Stammzelltransplantation erhielt der Patient von einem MUD-Spender mit unmanipulierten Knochenmark, die zweite von einem haploidenten Spender mit CD3/CD19 depletierten peripheren Blutstammzellen (PBSC). In beiden Fällen erfolgte keine Rekonstitution des Immunsystems. Erst als der Patient eine dritte Stammzelltransplantation mit TCR alpha/beta und CD19 depletierten PBSCs des Vaters erhielt, erfolgte eine sehr schnelle Immunrekonstitution.

Die GvHD war in den Fällen der TCR alpha/beta und CD19 Transplantation entgegen den Erwartungen nicht erhöht (Figur 2). Dabei ist zu berücksichtigen, dass nur die Haut von der GvHD betroffen war und die GvHD-Symptome nur temporär auftraten, obwohl keine Immunsuppression zur Behandlung gegeben wurde.

Grund für die schnelle Immunrekonstitution könnten die TCR gamma/delta Zellen sein, die bei einem TCR alpha/beta depletierten Zellpräparat im Transplantat enthalten sind, bei einem CD34 positiven Stammzelltransplantat aber nicht.

### Stammzell- und Knochenmarktransplantation

Bei der Knochenmarktransplantation wird dem Spender unter Vollnarkose ungefähr ein Liter Knochenmark-Blut-Gemisch aus dem Beckenknochen entnommen.

Bei der Stammzellentnahme aus dem Blut wird dem Spender über mehrere Tage ein körpereigener hormonähnlicher Stoff verabreicht, der die Produktion der Stammzellen und ihren Übertritt aus dem Knochenmark in den Blutkreislauf anregt. Die Methoden zur Vorbehandlung des Spenders und zur Entnahme von Knochenmarks- oder Blutstammzellen sind Stand der Technik und dem Fachmann bekannt.

### Ablauf

Das Ziel der Blutstammzelltransplantation besteht darin, den Empfänger mit einer gesunden Stammzellpopulation auszustatten, die sich zu Blutzellen ausdifferenzieren. Dadurch werden die defizienten oder pathologischen Zellen des Empfängers ersetzt (*Beers und Berkow 2000).* Bei der allogenen Transplantation stammt das Gewebe von einem gesunden Spender. Dies kann ein eineiiger Zwilling (identical sibling twin), eine HLA-identisches Geschwisterkind (identical sibling), ein nicht HLA-identisches Familienmitglied (mismatched related donor) ein (haploidentischer Spender) oder ein unverwandter HLA-kompatibeler Spender sein. Das Hauptziel der allogenen Transplantation ist es, das erkrankte oder defekte hämatopoietische System, wie z. B. das Knochenmark des Empfängers, vollständig durch ein gesundes, funktionierendes hämatopoetisches System (u. a. Immunsystem) zu ersetzen. Die Stammzelltransplantation kann aber auch mit autologen d. h. patienteneigenen Zellen erfolgen.

### Spender (IdSib, MUD, Haploidente Spender)

Als Spender der ersten Wahl gilt ein hinsichtlich der relevanten Histokompatibilitäts-Antigene HLA-A, B, C, DRB1 und DQB1 identisches Geschwister (Identical Sibling = IdSib). Dieses kann allerdings in nur ca 30 % gefunden werden, so dass stattdessen oft ein HLA-identischer unverwandter Spender (matched unrelated donor, MUD) gesucht werden muss (Ottinger et al., 2001). Da bei weitem nicht alle Histokompatibilitäts-Antigene bekannt sind und auch nur eine begrenzte Zahl von Allelen getestet werden können, muss beim identischen Fremdspender von einer schlechteren Übereinstimmung als beim Geschwisterspender ausgegangen werden.
Es bleibt immer noch ein beträchtlicher Teil ohne Spender. Für diese Patienten können verwandte Spender genutzt werden, die mit dem Empfänger nur in einem Haplotyp ihrer HLA-Allele übereinstimmen, also haploidentisch sind.

Transplantate von unverwandten Spendern (MUD) werden für hämatopoetische Stammzelltransplantationen am häufigsten verwendet (Blood 2003; 101(4): 1630-6).

### MUD: Unmanipuliertes Transplantat

Bei unmanipulierten Transplantaten im MUD Setting ist die GvHD die Hauptkomplikation. Schwere Formen der GvHD sind als lebensbedrohlich einzustufen und erfordern eine massive Therapie mit immunsppressiven Substanzen, für die Ansprechraten von ca. 40 % beschreiben werden (Vogelsang et al., 2003). Die akute GvHD Grad II-IV: V:33%; C: 51% und Grad III-IV: V:11.7%; C: 24,5% (Finke et al., Lancet, 2009).

Alternativ wurde CD34 angereichertes Transplantat im MUD Setting verwendet, um die GvHD zu reduzieren und die mit der notwendigen GvHD-Prophylaxe verwendeten Nebenwirkungen zu vermeiden. Nachteil ist die verzögerte Immunrekonstitution mit allen Folgen, wie bereits erwähnt.

### Eigentliche Transplantation

Die eigentliche Transplantation gliedert sich in zwei Phasen. Mit der Konditionierung durch Chemo- und/oder Strahlentherapie wird das Immunsystem des Empfängers zerstört, damit es das übertragene bzw. transplantierte Knochenmark bzw. die Stammzellen nicht abstößt. D. h., der Empfänger wird auf das Angehen des Transplantats vorbereitet. Je besser dies gelingt, desto geringer ist das Risiko eines Nichtanwachsens oder einer Abstoßung des Transplantates. Je nach Stärke der Konditionierung wird das Ziel verfolgt, im Patienten noch verbliebenen leukämischen oder bösartigen Zellen zu vernichten. Die Transplantation erfolgt am Tag 0 intravenös. Bis zum Anwachsen (engraftment) des Transplantats und Abklingen der unmittelbaren Toxizitäten verbleibt der Patient in der Regel auf einer speziell eingerichteten Station. Nach Anwachsen des Transplantats und Abklingen der unmittelbaren Toxizitäten ist während der ersten drei Monate eine enge Überwachung notwendig. Die Intensität der Überwachung hängt stark vom Spendertyp und den Komplikationen ab und geht kontinuierlich in die regelmäßige lebenslange Nachsorge über.

### Indikationen

Alle Indikationen, die eine allogene Stamzelltransplantation erforderlich machen, können mit der erfindungsgemäßen Zellpopulation bzw. pharmazeutischen Zusammensetzung behandelt werden. Alle schweren angeborenen und erworbenen malignen und nichtmaligenen Erkrankungen des hämatopoetischen Systems sind grundsätzlich Indikationen für eine allogene Stammzelltransplantation. Hinzu kommen maligne Erkrankungen, die auf eine Dosisintensivierung der Chemotherapie oder Strahlentherapie ansprechen.

Immunsuppressiva wie Ciclosporin, Kortikosteroide, Antimetabolite und monoklonale Antilymphozyten-Antikörper werden heute routinemäßig eingesetzt, um die GvHD besser kontrollieren zu können.

### Depletion von TCRα/β⁺ Zellen

Die Depletion von TCRα/β+ ist z. B. in Chaleff et al., Cytotherapy, 2007, 9, 746-754 oder wie im entsprechenden Protokoll der Miltenyi Biotec GmbH beschrieben.

### Kombinierte Depletion von TCRα/β⁺/ CD19⁺Zellen

Das Leukapherese-Produkt wird mit CliniMACS^{®} PBS/EDTA Buffer verdünnt (mit HSA bis zu einer Endkonzentration von 0.5% (w/v)) vor magnetischer Markierung. Das Leukaphereseprodukt wird bis zum 3-fachen Volumen des Leukaphereseprodukts verdünnt ohne ein Maximalvolumen von 600 ml zu überschreiten.

Die Zellen werden bei 200xg für 15 min. bei Raumtemperatur zentrifugiert (+19 °C to +25 °C). Der Überstand wird verworfen. Das optimale Gewicht für das Labelling ist 88 g (± 5 g). Das Pellet wird resuspendiert und das Gewicht bestimmt. Die Zellen werden mit CliniMACS^{®} TCRα/β-Biotin und mit CliniMACS^{®} CD19 Reagenz, ein Gefäß (7.5 ml) CliniMACS^{®} TCRα/β-Biotin und ein Gefäß (7.5 ml) CliniMACS^{®} CD19 gelabelt. Die Gefäße werden bei +2 °C to +8 °C gelagert und kalt verwendet. Zellen und Reagenzien werden gemischt, die Inkubationszeit beträgt 30 min bei 25 rpm und Raumtemperatur (+19 °C to +25 °C).

Leukapherese Produkt und Puffer werden gemischt und leicht bewegt, gefolgt von einer Zentrifugation bei 300xg für 15 min. ohne Bremse bei Raumtemperatur (+19 °C to +25 °C). Der Überstand wird verworfen. Das Pellet wird resuspendiert und gewaschen. Puffer wird hinzugefügt bis ca. 190 g Gewicht erreicht werden für das magnetische Labelling mit den TCRα/β-Biotin gelabelten Zellen mit dem CliniMACS^{®} Anti-Biotin Reagenz. Die bei +2 °C to +8 °C gekühlten (7.5 ml) CliniMACS^{®} Anti-Biotin Reagenzien werden den Zellen hinzugefügt, 30 min. inkubiert bei leichtem Bewegen der Zellen bei 25 rpm und Raumtemperatur (+19 °C to +25 °C).

Leukapherese Produkt und 500 ml Puffer werden leicht bewegt und gemischt, dann bei 300xg für 15 min. ohne Bremse bei Raumtemperatur (+19 °C to +25 °C) zentrifugiert. Der Überstand wird verworfen, das Pellet resuspendiert bis 150g erreicht sind. Es wird empfohlen, eine maximale Konzentration von 0,4x10⁹ Zellen pro ml einzuhalten.

Das Programm DEPLETION 3.1 auf dem CliniMACS^{® plus} Instrument wird gestartet und den Anweisungen gefolgt wie vom Hersteller Miltenyi Biotec GmbH angegeben. Nachdem die automatische Separation beendet ist, wird die Zellkonzentration bestimmt. Die Zellen sind nach der automatischen Separation TCR alpha/beta und CD19 mit ca. 3 bis 5 Logstufen depletiert. Das so erhaltene TCR alpha/beta und CD 19 depletierte Zellpräparat kann zur Transplantation verwendet werden, nachdem es in einer für die Transplanation geeigneten Lösung aufgenommen wurde. Derartige Lösungen sind dem Fachmann bekannt.

### Figuren

**Figur 1:** Immunrekonstitution nach Stammzelltransplantation bis zum Erreichen von > 200 Zellen/µl.

Dargestellt ist die Immunrekonstitution bis zum Erreichen von >200 Zellen/µl nach Transplantation von TCRαβ/CD19 depletierten peripheren Blutstammzellen (N=11) im Vergleich zu CD34 angereicherten peripheren Blutstammzellen (historische Kontrolle, N=13). Die Daten zur Immunrekonstitution nach Stammzelltransplantation mit CD34 angereicherten Zellen wurden der folgenden Publikation entnommen: Br J Haematol. 2001; 114: 422-32.

### Figur 2: GvHD nach Stammzelltransplantation

Inzidenz der akuten GvHD bei Patienten mit TCRαβ/CD19 depletierten haploidentischen Transplantaten. Vergleichsgruppen (historische Kontrollen): Patienten mit CD34 angereicherten haploidentischen Transplantaten und Patienten mit unmanipuliertem Knochenmark von identischen Fremdspendern und Methotrexat/CsA zur GvHD Prophylaxe. Die Daten der Vergleichsgruppen wurden aus Lang et al. 2007, Zeitschrift für Regenerative Medizin, Nr.1: 32-39 entnommen. "Grade" = Stadium

### Figur 3 (A und B): Analyse der T-Zell-Rezeptor β Repertoire Diversität zur Bestätigung der Thymus abhängigen T-Zell Rekonstitution

Exemplarisch dargestellt ist das Ergebnis der Analyse des TCRβ Repertoire Diversität durch CDR3 Spectratyping zur Messung der Thymus-abhängigen T-Zell Rekonstitution bei einem Patienten an Tag 12 (Figur 3A) und Tag 33 (Figur 3B). Die T-Zell-Rezeptor CDR3 Region ist die einzige nicht Keimbahn kodierte hypervariabele Region. Diese TCRαβ Region wird im Thymus u. a. durch Rekombination erzeugt. Die Methode ist in Bone Marrow Transplant. 2008 Oct; 42 Suppl 2: S54-9 beschrieben.

### Figur 4: T-Zell-Rezeptor Excision Circles (TRECs) im peripheren Blut zur Quantifizierung der aus dem Thymus stammenden T-Zellen

Exemplarisch dargestellt ist das Ergebnis der Bestimmung des T-Zell Rezeptor Excision Circles (TRECS) im peripheren Blut zur Bestätigung, dass T-Zellen nach Transplantation im Thymus produziert wurden. Diese Methode wurde in vielen Studien nach der Transplantation eingesetzt, um die Aktivität der Thymus abzuschätzen. Die Methode ist in Zhonghua Yi Xue Za Zhi. 2007 Aug 28;87(32):2265-7 beschrieben, "threshold cycle C(t)" = Grenzwertzyklus C(t), "not depleted" = nicht depletiert, "pre TX" = prä TX (Transplantation)

**Figur 5 (A, B und C):** Klinische Ergebnisse bei Kindern. Vergleichende Analyse der T-Zell-Regeneration eines Patienten.
1. Transplantat: Unmanipuliertes Knochenmark von MUD Spender (2008)
2. Transplantat: CD3/CD19 depletiertes PBSC der Mutter (2009)
3. Transplantat: TCRα/β/CD19 depletiertes PBSC des Vaters (9 Monate nach 2. Transplantation)

Die Graphen zeigen die Konzentration von (von links nach rechts) CD3 positiven Zellen (A), CD4 positiven Zellen (B) und CD8 positiven Zellen (C) zu verschienen Zeitpunkten nach Transplantation von Knochenmark (Zellen / Mikroliter).

### Beispiel: Transplantation

Es wurden elf Patienten transplantiert, fünf mit der Diagnose akute lymphatische Leukämie (ALL), drei Patienten mit der Diagnose akute myeloide Leukämie (AML), ein Patient mit der Diagnose Agranulocytose, ein Patient mit der Diagnose beta-Thalassenämie, ein Patient mit der Diagnose Inborn Error (HHS). Fünf der Patienten hatten bereits eine Stammzelltransplantation zuvor erhalten, drei weitere hatten bereits zwei bzw. drei Stammzelltransplantationen erhalten.

Bei keinem Patienten erfolgte eine Immunsuppression nach Transplantation.

### Beispiel: MUD Spender

Ein Patient mit beta-Thalassenämie, ein Patient mit ALL und ein Patient mit Inborn Error erhielt Spendermaterial von einem MUD-Spender mit einem TCR alpha/beta-depletierten und CD19-depletierten Zellpräparat.

### Beispiel: chronische GvHD

Ein Patient, der Material von einem haploidenten Spender erhielt entwickelte eine chronische GvHD mit mildem Verlauf.

Kein Patient, der Material von einem MUD-Spender erhielt entwickelte eine chronische GvHD.

## Patentansprüche

1. Pharmazeutische Zusammensetzung, aufweisend eine aus Knochenmark oder aus Blut erhaltbare Zellpopulation, wobei die Zellpopulation von TCR alpha/beta-positiven Zellen und von CD19-positiven Zellen depletiert ist und TCR gamma/delta-positive Zellen aufweist.

2. Pharmazeutische Zusammensetzung nach Anspruch 1, aufweisend einen pharmazeutisch akzeptablen Träger.

3. Verfahren zur Herstellung einer TCR gamma/delta-positive Zellen aufweisenden Zellpopulation aus Knochenmark oder Blut, aufweisend die - Depletion von TCR alpha/ beta-positiven und von CD19-positiven Zellen.

4. Verfahren nach Anspruch 3, wobei die Depletion von TCR alpha/beta positiven Zellen mittels eines Antikörpers oder eines Antigen-bindenden Fragments gegen TCR alpha/ beta erfolgt.

5. Verfahren nach Anspruch 3, wobei die Depletion von CD19-positiven Zellen mittels eines Antikörpers oder eines Antigen-bindenden Fragments gegen CD19 erfolgt.

6. Verwendung eines Antikörpers oder eines Antigen-bindenden Fragments gegen TCR alpha/beta und/oder eines Antikörpers oder eines Antigen-bindenden Fragments gegen CD19 zur Herstellung einer Zellpopulation in vitro, die TCR alpha/beta und CD19 depletiert ist und TCR gamma/delta-positive Zellen aufweist.

7. Verwendung eines Kits zur Herstellung einer Zellpopulation, die TCR alpha/beta depletiert und CD19 depletiert ist und TCR gamma/delta-positive Zellen aufweist, aufweisend einen Antikörper oder ein Antigen-bindendes Fragment gegen TCR alpha/beta und einen Antikörper oder ein Antigen-bindendes Fragment gegen CD19.

## Claims

1. A pharmaceutical composition comprising a cell population derivable from bone marrow or from blood, wherein the cell population is depleted of TCR alpha/beta positive cells and of CD19 positive cells and comprises TCR gamma/delta positive cells.

2. The pharmaceutical composition of claim 1 comprising a pharmaceutically acceptable carrier.

3. A method for producing a cell population comprising TCR gamma/delta positive cells from bone marrow or blood, comprising the depletion of TCR alpha/beta positive cells and of CD19 positive cells.

4. The method of claim 3, wherein the depletion of TCR alpha/beta positive cells is performed using an antibody or an antigen-binding fragment against TCR alpha/beta.

5. The method of claim 3, wherein the depletion of CD19 positive cells is performed using an antibody or an antigen-binding fragment against CD19.

6. Use of an antibody or an antigen-binding fragment against TCR alpha/beta and/or of an antibody or an antigen binding-fragment against CD19 for preparing a cell population in vitro that is depleted of TCR alpha/beta and CD19 and comprises TCR gamma/delta positive cells.

7. Use of a kit for preparing a cell population that is depleted of TCR alpha/beta and CD19 and comprises TCR gamma/delta positive cells comprising an antibody or an antigen-binding fragment against TCR alpha/beta and an antibody or an antigen-binding fragment against CD19.

## Revendications

1. Composition pharmaceutique, comprenant une population cellulaire pouvant être obtenue à partir de moelle osseuse ou à partir de sang, la population cellulaire étant appauvrie en cellules TCR alpha/bêta-positives et en cellules CD19-positives, et comprenant des cellules TCR gamma/delta-positives.

2. Composition pharmaceutique selon la revendication 1, comprenant un véhicule pharmaceutiquement acceptable.

3. Procédé de fabrication d'une population cellulaire comprenant des cellules TCR gamma/delta-positives à partir de moelle osseuse ou de sang, comprenant l'appauvrissement en cellules TCR alpha/bêta-positives et en cellules CD19-positives.

4. Procédé selon la revendication 3, dans lequel l'appauvrissement en cellules TCR alpha/bêta-positives a lieu au moyen d'un anticorps ou d'un fragment de liaison à l'antigène contre TCR alpha/bêta.

5. Procédé selon la revendication 3, dans lequel l'appauvrissement en cellules CD19-positives a lieu au moyen d'un anticorps ou d'un fragment de liaison à l'antigène contre CD19.

6. Utilisation d'un anticorps ou d'un fragment de liaison à l'antigène contre TCR alpha/bêta et/ou d'un anticorps ou d'un fragment de liaison à l'antigène contre CD19 pour la fabrication d'une population cellulaire in vitro, qui est appauvrie en TCR alpha/bêta et CD19 et comprend des cellules TCR gamma/delta-positives.

7. Utilisation d'un kit pour la fabrication d'une population cellulaire qui est appauvrie en TCR alpha/bêta et en CD19 et comprend des cellules TCR gamma/delta-positives, comprenant un anticorps ou un fragment de liaison à l'antigène contre TCR alpha/bêta et un anticorps ou un fragment de liaison à l'antigène contre CD19.


### SEQUENCE LISTING

<110> Miltenyi Biotec GmbH
<120> TCRalpha/beta-depletierte Zellseparationen
<130> MIL017WO
<150> DE102011001380.6
   <151> 2011-03-17
<150> EP11168949.3
   <151> 2011-06-07
<160> 14
<170> PatentIn version 3.5
<210> 1
   <211> 556
   <212> PRT
   <213> Homo sapiens
<400> 1
<210> 2
   <211> 1965
   <212> DNA
   <213> homo sapiens
<400> 2
   aggcccctgc ctgccccagc atcccctgcg cgaagctggg tgccccggag agtctgacca      60
   ccatgccacc tcctcgcctc ctcttcttcc tcctcttcct cacccccatg gaagtcaggc     120
   ccgaggaacc tctagtggtg aaggtggaag agggagataa cgctgtgctg cagtgcctca     180
   aggggacctc agatggcccc actcagcagc tgacctggtc tcgggagtcc ccgcttaaac     240
   ccttcttaaa actcagcctg gggctgccag gcctgggaat ccacatgagg cccctggcca     300
   tctggctttt catcttcaac gtctctcaac agatgggggg cttctacctg tgccagccgg     360
   ggcccccctc tgagaaggcc tggcagcctg gctggacagt caatgtggag ggcagcgggg     420
   agctgttccg gtggaatgtt tcggacctag gtggcctggg ctgtggcctg aagaacaggt     480
   cctcagaggg ccccagctcc ccttccggga agctcatgag ccccaagctg tatgtgtggg     540
   ccaaagaccg ccctgagatc tgggagggag agcctccgtg tctcccaccg agggacagcc 600
   tgaaccagag cctcagccag gacctcacca tggcccctgg ctccacactc tggctgtcct 660
   gtggggtacc ccctgactct gtgtccaggg gccccctctc ctggacccat gtgcacccca 720
   aggggcctaa gtcattgctg agcctagagc tgaaggacga tcgcccggcc agagatatgt 780
   gggtaatgga gacgggtctg ttgttgcccc gggccacagc tcaagacgct ggaaagtatt 840
   attgtcaccg tggcaacctg accatgtcat tccacctgga gatcactgct cggccagtac 900
   tatggcactg gctgctgagg actggtggct ggaaggtctc agctgtgact ttggcttatc 960
   tgatcttctg cctgtgttcc cttgtgggca ttcttcatct tcaaagagcc ctggtcctga 1020
   ggaggaaaag aaagcgaatg actgacccca ccaggagatt cttcaaagtg acgcctcccc 1080
   caggaagcgg gccccagaac cagtacggga acgtgctgtc tctccccaca cccacctcag 1140
   gcctcggacg cgcccagcgt tgggccgcag gcctgggggg cactgccccg tcttatggaa 1200
   acccgagcag cgacgtccag gcggatggag ccttggggtc ccggagcccg ccgggagtgg 1260
   gcccagaaga agaggaaggg gagggctatg aggaacctga cagtgaggag gactccgagt 1320
   tctatgagaa cgactccaac cttgggcagg accagctctc ccaggatggc agcggctacg 1380
   agaaccctga ggatgagccc ctgggtcctg aggatgaaga ctccttctcc aacgctgagt 1440
   cttatgagaa cgaggatgaa gagctgaccc agccggtcgc caggacaatg gacttcctga 1500
   gccctcatgg gtcagcctgg gaccccagcc gggaagcaac ctccctgggg tcccagtcct 1560
   atgaggatat gagaggaatc ctgtatgcag ccccccagct ccgctccatt cggggccagc 1620
   ctggacccaa tcatgaggaa gatgcagact cttatgagaa catggataat cccgatgggc 1680
   cagacccagc ctggggagga gggggccgca tgggcacctg gagcaccagg tgatcctcag 1740
   gtggccagcc tggatctcct caagtcccca agattcacac ctgactctga aatctgaaga 1800
   cctcgagcag atgatgccaa cctctggagc aatgttgctt aggatgtgtg catgtgtgta 1860
   agtgtgtgtg tgtgtgtgtg tgtgtataca tgccagtgac acttccagtc ccctttgtat 1920
   tccttaaata aactcaatga gctcttccaa tcctaaaaaa aaaaa 1965
<210> 3
   <211> 7828
   <212> DNA
   <213> Homo sapiens
<400> 3
<210> 4
   <211> 142
   <212> PRT
   <213> Homo sapiens
<400> 4
   Pro Asn Ile Gln Asn Pro Asp Pro Ala Val Tyr Gln Leu Arg Asp Ser
   1               5                   10                  15
   Lys Ser Ser Asp Lys Ser Val Cys Leu Phe Thr Asp Phe Asp Ser Gln
               20                  25                  30
   Thr Asn Val Ser Gln Ser Lys Asp Ser Asp Val Tyr Ile Thr Asp Lys
           35                  40                  45
   Thr Val Leu Asp Met Arg Ser Met Asp Phe Lys Ser Asn Ser Ala Val
       50                  55                  60
   Ala Trp Ser Asn Lys Ser Asp Phe Ala Cys Ala Asn Ala Phe Asn Asn
   65                  70                  75                  80
   Ser Ile Ile Pro Glu Asp Thr Phe Phe Pro Ser Pro Glu Ser Ser Cys
                   85                  90                  95
   Asp Val Lys Leu Val Glu Lys Ser Phe Glu Thr Asp Thr Asn Leu Asn
               100                 105                 110
   Phe Gln Asn Leu Ser Val Ile Gly Phe Arg Ile Leu Leu Leu Lys Val
           115                 120                 125
   Ala Gly Phe Asn Leu Leu Met Thr Leu Arg Leu Trp Ser Ser
       130                 135                 140
<210> 5
   <211> 426
   <212> DNA
   <213> Homo sapiens
<400> 5
   ccaaatatcc agaaccctga ccctgccgtg taccagctga gagactctaa atccagtgac      60
   aagtctgtct gcctattcac cgattttgat tctcaaacaa atgtgtcaca aagtaaggat     120
   tctgatgtgt atatcacaga caaaactgtg ctagacatga ggtctatgga cttcaagagc     180
   aacagtgctg tggcctggag caacaaatct gactttgcat gtgcaaacgc cttcaacaac     240
   agcattattc cagaagacac cttcttcccc agcccagaaa gttcctgtga tgtcaagctg     300
   gtcgagaaaa gctttgaaac agatacgaac ctaaactttc aaaacctgtc agtgattggg     360
   ttccgaatcc tcctcctgaa agtggccggg tttaatctgc tcatgacgct gcggctgtgg     420
   tccagc                                                                426
<210> 6
   <211> 3430
   <212> DNA
   <213> Homo sapiens
<400> 6
   ttcccgtata aagcatgaga ccgtgacttg ccagccccac agagccccgc ccttgtccat      60
   cactggcatc tggactccag cctgggttgg ggcaaagagg gaaatgagat catgtcctaa     120
   ccctgatcct cttgtcccac agatatccag aaccctgacc ctgccgtgta ccagctgaga     180
   gactctaaat ccagtgacaa gtctgtctgc ctattcaccg attttgattc tcaaacaaat     240
   gtgtcacaaa gtaaggattc tgatgtgtat atcacagaca aaactgtgct agacatgagg     300
   tctatggact tcaagagcaa cagtgctgtg gcctggagca acaaatctga ctttgcatgt     360
   gcaaacgcct tcaacaacag cattattcca gaagacacct tcttccccag cccaggtaag     420
   ggcagctttg gtgccttcgc aggctgtttc cttgcttcag gaatggccag gttctgccca     480
   gagctctggt caatgatgtc taaaactcct ctgattggtg gtctcggcct tatccattgc     540
   caccaaaacc ctctttttac taagaaacag tgagccttgt tctggcagtc cagagaatga     600
   cacgggaaaa aagcagatga agagaaggtg gcaggagagg gcacgtggcc cagcctcagt     660
   ctctccaact gagttcctgc ctgcctgcct ttgctcagac tgtttgcccc ttactgctct     720
   tctaggcctc attctaagcc ccttctccaa gttgcctctc cttatttctc cctgtctgcc     780
   aaaaaatctt tcccagctca ctaagtcagt ctcacgcagt cactcattaa cccaccaatc     840
   actgattgtg ccggcacatg aatgcaccag gtgttgaagt ggaggaatta aaaagtcaga     900
   tgaggggtgt gcccagagga agcaccattc tagttggggg agcccatctg tcagctggga     960
   aaagtccaaa taacttcaga ttggaatgtg ttttaactca gggttgagaa aacagccacc    1020
   ttcaggacaa aagtcaggga agggctctct gaagaaatgc tacttgaaga taccagccct    1080
   accaagggca gggagaggac cctatagagg cctgggacag gagctcaatg agaaaggaga    1140
   agagcagcag gcatgagttg aatgaaggag gcagggccgg gtcacagggc cttctaggcc    1200
   atgagagggt agacagtatt ctaagtacgc cagaaagctg ttgatcggct tcaagcaggg    1260
   aagggacacc taatttgctt ttcttttctt tttttttttt tttttttttt tttttttgag    1320
   atggagtttt gctcttgttg cccaggctgg agtgcaatgg tgcatcttgg ctcactacaa    1380
   gcctctgcct cccaggttca agtgattctc ctgcctcagc ctcccaagta gctgggatta    1440
   caggcaccca ccaccatgcc cggctaattt tttgtatttt tagtagagac agggtttcac    1500
   tatgttggcc aggctggtct cgaactcctg acctcaggtg atccacccgc ttcagcctcc    1560
   caaagtgctg ggattacagg cgtgagccac cacacccggc ctgcttttct taaagatcaa 1620
   tctgagtgct gtacggagag tgggttgtaa gccaagagta gaagcagaaa gggagcagtt 1680
   gcagcagaga gatgatggag gcctgggcac ggtggtggca gggaggtaac caacaccatt 1740
   caggtttcaa aggtagaacc atgcagggat gagaaagcaa agaggggatc aaggaaggca 1800
   gctggatttt ggcctgagca gctgagtcaa tgatagtgcc gtttactaag aagaaaccaa 1860
   ggaaaaaatt tggggtgcag ggatcaaaac tttttggaac atatgaaagt acgtgtttat 1920
   actctttatg gcccttgtca ctatgtatgc ctcgctgcct cattggactc tagaatgaag 1980
   ccaggcagag cagggtctat gtgtgatggc acatgtggcc agggtcatgc agacatgtac 2040
   tttgtacaaa cagtgtatat tgagtaaata gaaatggtgt ccaggagccg aggtatcgtc 2100
   ctgccagggc caggggctct ccctagcagg tgctcatatg ctgtaagttc cctccagatc 2160
   tctccacaag gaggcatgga aaggctgtag ttgttcacct gcccaagaac taggaggtct 2220
   ggggtgggag agtcagcctg ctctggatgc tgaaagaatg tctgtttttc cttttagaaa 2280
   gttcctgtga tgtcaagctg gtcgagaaaa gctttgaaac aggtaagaca ggggtctagc 2340
   ctgggtttgc acaggattgc cgaagtgatg aacccgcaat aaccctgcct ggatgaggga 2400
   gtgggaagaa attagtagat gtgggaatga atgatgagga atggaaacag cggttcaaga 2460
   cctgcccaga gctgggtggg gtctctcctg aatccctctc accgtctctg actttccgtt 2520
   ctaagcactt tgaggatgag tttctagctt caatagacca aggactctct cctaggcctc 2580
   tgtattcctt tcaacagctc cactgtcaag agagccagag agagcttctg ggtggcccag 2640
   ctgtgaaatt tctgagtccc ttagggatag ccctaaacga accagatcat cctgaggaca 2700
   gccaagaggt tttgccttct ttcaagacaa gcaacagtac tcacataggc tgtgggcaat 2760
   ggtcctgtct ctcaagaatc ccctgccact cctcacaccc accctgggcc catattcatt 2820
   tccatttgag ttgttcttat tgagtcatcc ttcctgtggc agcggaactc actaaggggc 2880
   ccatctggac ccgaggtatt gtgaagataa attctgagca cctaccccat ccccagaagg 2940
   gctcagaaat aaaataagag ccaagtctag tcggtgtttc ctgtcttgaa acacaatact 3000
   gttggccctg gaagaatgca cagaatctgt ttgtaagggg atatgcacag aagctgcaag 3060
   ggacaggagg tgcaggagct gcaggcctcc cccacccagc ctgctctgcc ttggggaaaa 3120
   ccgtgggtgt gtcctgcagg ccatgcaggc ctgggacatg caagcccata accgctgtgg 3180
   cctcttggtt ttacagatac gaacctaaac tttcaaaacc tgtcagtgat tgggttccga 3240
   atcctcctcc tgaaagtggc cgggtttaat ctgctcatga cgctgcggct gtggtccagc 3300
   tgaggtgagg ggccttgaag ctgggagtgg ggtttaggga cgcgggtctc tgcgtgcatc 3360
   ctaagctctg agagcaaacc tccctgcagg gtcttgcttt taagtccaaa gcctgagccc 3420
   accaaactct 3430
<210> 7
   <211> 177
   <212> PRT
   <213> Homo sapiens
<400> 7
   Glu Asp Leu Asn Lys Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Phe Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe Tyr Gly Leu Ser Glu Asn Asp Glu Trp Thr Gln Asp
               100                 105                 110
   Arg Ala Lys Pro Val Thr Gln Ile Val Ser Ala Glu Ala Trp Gly Arg
           115                 120                 125
   Ala Asp Cys Gly Phe Thr Ser Val Ser Tyr Gln Gln Gly Val Leu Ser
       130                 135                 140
   Ala Thr Ile Leu Tyr Glu Ile Leu Leu Gly Lys Ala Thr Leu Tyr Ala
   145                 150                 155                 160
   Val Leu Val Ser Ala Leu Val Leu Met Ala Met Val Lys Arg Lys Asp
                   165                 170                 175
   Phe
<210> 8
   <211> 531
   <212> DNA
   <213> Homo sapiens
<400> 8
   gaggacctga acaaggtgtt cccacccgag gtcgctgtgt ttgagccatc agaagcagag      60
   atctcccaca cccaaaaggc cacactggtg tgcctggcca caggcttctt ccccgaccac     120
   gtggagctga gctggtgggt gaatgggaag gaggtgcaca gtggggtcag cacagacccg     180
   cagcccctca aggagcagcc cgccctcaat gactccagat actgcctgag cagccgcctg     240
   agggtctcgg ccaccttctg gcagaacccc cgcaaccact tccgctgtca agtccagttc     300
   tacgggctct cggagaatga cgagtggacc caggataggg ccaaacccgt cacccagatc     360
   gtcagcgccg aggcctgggg tagagcagac tgtggcttta cctcggtgtc ctaccagcaa     420
   ggggtcctgt ctgccaccat cctctatgag atcctgctag ggaaggccac cctgtatgct     480
   gtgctggtca gcgcccttgt gttgatggcc atggtcaaga gaaaggattt c              531
<210> 9
   <211> 2042
   <212> DNA
   <213> Homo sapiens
<400> 9
   tgcatcctag ggacagcata gaaaggaggg gcaaagtgga gagagagcaa cagacactgg      60
   gatggtgacc ccaaaacaat gagggcctag aatgacatag ttgtgcttca ttacggccca     120
   ttcccagggc tctctctcac acacacagag cccctaccag aaccagacag ctctcagagc     180
   aaccctggct ccaacccctc ttccctttcc agaggacctg aacaaggtgt tcccacccga     240
   ggtcgctgtg tttgagccat cagaagcaga gatctcccac acccaaaagg ccacactggt     300
   gtgcctggcc acaggcttct tccccgacca cgtggagctg agctggtggg tgaatgggaa     360
   ggaggtgcac agtggggtca gcacggaccc gcagcccctc aaggagcagc ccgccctcaa     420
   tgactccaga tactgcctga gcagccgcct gagggtctcg gccaccttct ggcagaaccc     480
   ccgcaaccac ttccgctgtc aagtccagtt ctacgggctc tcggagaatg acgagtggac     540
   ccaggatagg gccaaacccg tcacccagat cgtcagcgcc gaggcctggg gtagagcagg     600
   tgagtggggc ctggggagat gcctggagga gattaggtga gaccagctac cagggaaaat     660
   ggaaagatcc aggtagcaga caagactaga tccaaaaaga aaggaaccag cgcacaccat     720
   gaaggagaat tgggcacctg tggttcattc ttctcccaga ttctcagccc aacagagcca     780
   agcagctggg tcccctttct atgtggcctg tgtaactctc atctgggtgg tgccccccat     840
   ccccctcagt gctgccacat gccatggatt gcaaggacaa tgtggctgac atctgcatgg     900
   cagaagaaag gaggtgctgg gctgtcagag gaagctggtc tgggcctggg agtctgtgcc     960
   aactgcaaat ctgactttac ttttaattgc ctatgaaaat aaggtctctc atttattttc    1020
   ctctccctgc tttctttcag actgtggctt tacctcgggt aagtaagccc ttccttttcc    1080
   tctccctctc tcatggttct tgacctagaa ccaaggcatg aagaactcac agacactgga    1140
   gggtggaggg tgggagagac cagagctacc tgtgcacagg tacccacctg tccttcctcc     1200
   gtgccaacag tgtcctacca gcaaggggtc ctgtctgcca ccatcctcta tgagatcctg     1260
   ctagggaagg ccaccctgta tgctgtgctg gtcagcgccc ttgtgttgat ggccatggta     1320
   agcaggaggg caggatgggg ccagcaggct ggaggtgaca cactgacacc aagcacccag     1380
   aagtatagag tccctgccag gattggagct gggcagtagg gagggaagag atttcattca     1440
   ggtgcctcag aagataactt gcacctctgt aggatcacag tggaagggtc atgctgggaa     1500
   ggagaagctg gagtcaccag aaaacccaat ggatgttgtg atgagcctta ctatttgtgt     1560
   ggtcaatggg ccctactact ttctctcaat cctcacaact cctggctctt aataaccccc     1620
   aaaactttct cttctgcagg tcaagagaaa ggatttctga aggcagccct ggaagtggag     1680
   ttaggagctt ctaacccgtc atggtttcaa tacacattct tcttttgcca gcgcttctga     1740
   agagctgctc tcacctctct gcatcccaat agatatcccc ctatgtgcat gcacacctgc     1800
   acactcacgg ctgaaatctc cctaacccag gggaccttta gcatgcctaa gtgactaaac     1860
   caataaaaat gttctggtct ggcctgactc tgacttgtga atgtctggat agctccttgg     1920
   ctgtctctga actccctgtg actctcccca ttcagtcagg atagaaacaa gaggtattca     1980
   aggaaaatgc agactcttca cgtaagaggg atgaggggcc caccttgaga tcaatagcag     2040
   aa                                                                    2042
<210> 10
   <211> 179
   <212> PRT
   <213> Homo sapiens
<400> 10
   Glu Asp Leu Lys Asn Val Phe Pro Pro Glu Val Ala Val Phe Glu Pro
   1               5                   10                  15
   Ser Glu Ala Glu Ile Ser His Thr Gln Lys Ala Thr Leu Val Cys Leu
               20                  25                  30
   Ala Thr Gly Phe Tyr Pro Asp His Val Glu Leu Ser Trp Trp Val Asn
           35                  40                  45
   Gly Lys Glu Val His Ser Gly Val Ser Thr Asp Pro Gln Pro Leu Lys
       50                  55                  60
   Glu Gln Pro Ala Leu Asn Asp Ser Arg Tyr Cys Leu Ser Ser Arg Leu
   65                  70                  75                  80
   Arg Val Ser Ala Thr Phe Trp Gln Asn Pro Arg Asn His Phe Arg Cys
                   85                  90                  95
   Gln Val Gln Phe Tyr Gly Leu Ser Glu Asn Asp Glu Trp Thr Gln Asp
   100 105 110
   Arg Ala Lys Pro Val Thr Gln Ile Val Ser Ala Glu Ala Trp Gly Arg
   115 120 125
   Ala Asp Cys Gly Phe Thr Ser Glu Ser Tyr Gln Gln Gly Val Leu Ser
   130 135 140
   Ala Thr Ile Leu Tyr Glu Ile Leu Leu Gly Lys Ala Thr Leu Tyr Ala
   145 150 155 160
   Val Leu Val Ser Ala Leu Val Leu Met Ala Met Val Lys Arg Lys Asp
   165 170 175
   Ser Arg Gly
<210> 11
   <211> 178
   <212> PRT
   <213> Homo sapiens
<400> 11
   Asp Leu Lys Asn Val Phe Pro Pro Glu Val Ala Val Phe Glu Pro Ser
   1 5 10 15
   Glu Ala Glu Ile Ser His Thr Gln Lys Ala Thr Leu Val Cys Leu Ala
   20 25 30
   Thr Gly Phe Tyr Pro Asp His Val Glu Leu Ser Trp Trp Val Asn Gly
   35 40 45
   Lys Glu Val His Ser Gly Val Ser Thr Asp Pro Gln Pro Leu Lys Glu
   50 55 60
   Gln Pro Ala Leu Asn Asp Ser Arg Tyr Cys Leu Ser Ser Arg Leu Arg
   65 70 75 80
   Val Ser Ala Thr Phe Trp Gln Asn Pro Arg Asn His Phe Arg Cys Gln
   85 90 95
   Val Gln Phe Tyr Gly Leu Ser Glu Asn Asp Glu Trp Thr Gln Asp Arg
   100 105 110
   Ala Lys Pro Val Thr Gln Ile Val Ser Ala Glu Ala Trp Gly Arg Ala
   115 120 125
   Asp Cys Gly Phe Thr Ser Glu Ser Tyr Gln Gln Gly Val Leu Ser Ala
       130                 135                 140
   Thr Ile Leu Tyr Glu Ile Leu Leu Gly Lys Ala Thr Leu Tyr Ala Val
   145                 150                 155                 160
   Leu Val Ser Ala Leu Val Leu Met Ala Met Val Lys Arg Lys Asp Ser
                   165                 170                 175
   Arg Gly
<210> 12
   <211> 537
   <212> DNA
   <213> Homo sapiens
<400> 12
   gaggacctga aaaacgtgtt cccacccgag gtcgctgtgt ttgagccatc agaagcagag      60
   atctcccaca cccaaaaggc cacactggtg tgcctggcca caggcttcta ccccgaccac     120
   gtggagctga gctggtgggt gaatgggaag gaggtgcaca gtggggtcag cacagacccg     180
   cagcccctca aggagcagcc cgccctcaat gactccagat actgcctgag cagccgcctg     240
   agggtctcgg ccaccttctg gcagaacccc cgcaaccact tccgctgtca agtccagttc     300
   tacgggctct cggagaatga cgagtggacc caggataggg ccaaacctgt cacccagatc     360
   gtcagcgccg aggcctgggg tagagcagac tgtggcttca cctccgagtc ttaccagcaa     420
   ggggtcctgt ctgccaccat cctctatgag atcttgctag ggaaggccac cttgtatgcc     480
   gtgctggtca gtgccctcgt gctgatggcc atggtcaaga gaaaggattc cagaggc       537
<210> 13
   <211> 534
   <212> DNA
   <213> Homo sapiens
<400> 13
   gacctgaaaa acgtgttccc acccgaggtc gctgtgtttg agccatcaga agcagagatc 60
   tcccacaccc aaaaggccac actggtgtgc ctggccacag gcttctaccc cgaccacgtg 120
   gagctgagct ggtgggtgaa tgggaaggag gtgcacagtg gggtcagcac agacccgcag 180
   cccctcaagg agcagcccgc cctcaatgac tccagatact gcctgagcag ccgcctgagg 240
   gtctcggcca ccttctggca gaacccccgc aaccacttcc gctgtcaagt ccagttctac 300
   gggctctcgg agaatgacga gtggacccag gatagggcca aacctgtcac ccagatcgtc 360
   agcgccgagg cctggggtag agcagactgt ggcttcacct ccgagtctta ccagcaaggg 420
   gtcctgtctg ccaccatcct ctatgagatc ttgctaggga aggccacctt gtatgccgtg 480
   ctggtcagtg ccctcgtgct gatggccatg gtcaagagaa aggattccag aggc 534
<210> 14
   <211> 2008
   <212> DNA
   <213> Homo sapiens
<400> 14
   atggcgtagt ccccaaagaa cgaggaccta gtaacataat tgtgcttcat tatggtcctt 60
   tcccggcctt ctctctcaca catacacaga gcccctacca ggaccagaca gctctcagag 120
   caaccctagc cccattacct cttccctttc cagaggacct gaaaaacgtg ttcccacccg 180
   aggtcgctgt gtttgagcca tcagaagcag agatctccca cacccaaaag gccacactgg 240
   tgtgcctggc cacaggcttc taccccgacc acgtggagct gagctggtgg gtgaatggga 300
   aggaggtgca cagtggggtc agcacagacc cgcagcccct caaggagcag cccgccctca 360
   atgactccag atactgcctg agcagccgcc tgagggtctc ggccaccttc tggcagaacc 420
   cccgcaacca cttccgctgt caagtccagt tctacgggct ctcggagaat gacgagtgga 480
   cccaggatag ggccaaacct gtcacccaga tcgtcagcgc cgaggcctgg ggtagagcag 540
   gtgagtgggg cctggggaga tgcctggagg agattaggtg agaccagcta ccagggaaaa 600
   tggaaagatc caggtagcgg acaagactag atccagaaga aagccagagt ggacaaggtg 660
   ggatgatcaa ggttcacagg gtcagcaaag cacggtgtgc acttccccca ccaagaagca 720
   tagaggctga atggagcacc tcaagctcat tcttccttca gatcctgaca ccttagagct 780
   aagctttcaa gtctccctga ggaccagcca tacagctcag catctgagtg gtgtgcatcc 840
   cattctcttc tggggtcctg gtttcctaag atcatagtga ccacttcgct ggcactggag 900
   cagcatgagg gagacagaac cagggctatc aaaggaggct gactttgtac tatctgatat 960
   gcatgtgttt gtggcctgtg agtctgtgat gtaaggctca atgtccttac aaagcagcat 1020
   tctctcatcc atttttcttc ccctgttttc tttcagactg tggcttcacc tccggtaagt 1080
   gagtctctcc tttttctctc tatctttcgc cgtctctgct ctcgaaccag ggcatggaga 1140
   atccacggac acaggggcgt gagggaggcc agagccacct gtgcacaggt acctacatgc 1200
   tctgttcttg tcaacagagt cttaccagca aggggtcctg tctgccacca tcctctatga 1260
   gatcttgcta gggaaggcca ccttgtatgc cgtgctggtc agtgccctcg tgctgatggc 1320
   catggtaagg aggagggtgg gatagggcag atgatggggg caggggatgg aacatcacac 1380
   atgggcataa aggaatctca gagccagagc acagcctaat atatcctatc acctcaatga 1440
   aaccataatg aagccagact ggggagaaaa tgcagggaat atcacagaat gcatcatggg 1500
   aggatggaga caaccagcga gccctactca aattaggcct cagagcccgc ctcccctgcc 1560
   ctactcctgc tgtgccatag cccctgaaac cctgaaaatg ttctctcttc cacaggtcaa     1620
   gagaaaggat tccagaggct agctccaaaa ccatcccagg tcattcttca tcctcaccca     1680
   ggattctcct gtacctgctc ccaatctgtg ttcctaaaag tgattctcac tctgcttctc     1740
   atctcctact tacatgaata cttctctctt ttttctgttt ccctgaagat tgagctccca     1800
   acccccaagt acgaaatagg ctaaaccaat aaaaaattgt gtgttgggcc tggttgcatt     1860
   tcaggagtgt ctgtggagtt ctgctcatca ctgacctatc ttctgattta gggaaagcag     1920
   cattcgcttg gacatctgaa gtgacagccc tctttctctc cacccaatgc tgctttctcc     1980
   tgttcatcct gatggaagtc tcaacaca                                        2008